Europäisches Patentamt

European Patent Office

Office européen des brevets

(19)

(11) Publication number: **0 140 495 B1**

# EUROPEAN PATENT SPECIFICATION

(45) Date of publication of patent specification: **03.04.91**  (51) Int. Cl.⁵: **G01N 33/96**

(21) Application number: **84305406.5**

(22) Date of filing: **08.08.84**

(54) **Optically clear serum triglyceride compositions.**

(30) Priority: **22.08.83 US 524941**

(43) Date of publication of application:
**08.05.85 Bulletin 85/19**

(45) Publication of the grant of the patent:
**03.04.91 Bulletin 91/14**

(84) Designated Contracting States:
**BE CH DE FR GB IT LI NL SE**

(56) References cited:
**EP-A- 0 082 587
US-A- 3 955 925
US-A- 4 011 045
US-A- 4 045 176**

(73) Proprietor: **TECHNICON INSTRUMENTS COR-
PORATION(a Delaware corporation)
511 Benedict Avenue
Tarrytown, New York 10591-6097(US)**

(72) Inventor: **Buhl, Steven N.
4 South Delaware Drive
Nyack New York 10960(US)**

(74) Representative: **Wain, Christopher Paul et al
A.A. THORNTON & CO. Northumberland
House 303-306 High Holborn
London WC1V 7LE(GB)**

## Description

This invention relates to a method of making a stable, optically clear, triglyceride-containing serum especially useful as a standard or reference solution.

Reliable results in any chemical analytical determination of various blood serum components depend upon the continuous evaluation of the test procedure, equipment and reagents being used. This is accomplished by calibration of the instruments with standard solutions and the use of controls concurrent with the analysis to be performed (see, generally, U.S. Patent No. 4,158,544). These controls or standards contain known amounts of the material being analyzed, such as triglyceride. The level of triglyceride in normal human blood is critical, as it is generally recognized that high concentrations are related to coronary artery disease. It is, therefore, necessary to provide controls and standards containing normal and elevated triglyceride levels, to avoid discrepancies between various analytical procedures. Thus, accurate results in the use of instruments, particularly in the case of highly automated instruments, depend upon the strict standardization of the biochemical determinations being made. Substantial work has gone toward providing triglyceride-rich fractions from various sources in order to enhance the triglyceride levels of human and animal sera which are thereby made available for use as such calibrators or controls (see, for example, U.S. Patent No. 3,764,556).

The use of human sera in the preparation of chemical standards and controls for the assay of human serum constituents is well known, and they are usually provided as lyophilized materials to be reconsituted at the time of use. However, these sera are often highly turbid, both before lyophilization and after reconstitution. Even sera which are clear prior to lyophilization are sometimes found to be highly turbid upon reconstitution. This turbidity in serum and plasma samples is believed to be due to the presence of proteins or of lipids, which are primarily composed of triglycerides. Turbidity in lyophilized biological products has been shown to correlate with triglyceride level (see Buhl, et al, Clin. Chem. 27 :1076, abstract no. 263 (1981)). This turbidity of the serum affects the optical density read by instruments used in making these clinical determinations and, as such, interfere with the accuracy of the measurements reported. Chemical substances of various sorts have been used in attempts to reduce turbidity in such serum samples.

For example, U.S. Patent No. 3,853,465 discloses the reduction of serum turbidity by treating the serum with a polyoxyethylated lauric acid which remains in the serum even during use in an assay procedure.

More recently, U.S. Patent No. 3,955,925 has described a procedure for reducing serum turbidity by precipitating chylomicrons, unstable very low density lipoproteins and low density lipoproteins, from human serum. The process includes mixing a human serum sample with a metal cation and thereafter adding a polysulphate derivative which precipitates lipoprotein polysulphate complexes containing the constituents to be removed. A precipitating agent such as oxalate is then added to precipitate the remaining metal cation from the clear supernatant, which is then stored for use. This process requires the use of human serum for preparation of calibrators or controls. The triglyceride levels of the human serum, which are reduced by this precipitation, must be restored to desired levels by the addition of soluble derivatives, such as trichloroacetate triglyceride of glycerol triacetate, or by the addition of stable lyophilizable lipoproteins from animal sources, such as bovine lipoprotein fractions containing triglycerides, in order to provide a suitable calibrator or control preparation. U.S. Patent 4, 045, 176 further describes the preparation of standards and controls by the addition of human high density lipoproteins or non-primate lipoproteins to human serum. As in U.S. Patent 3955925, it is indicated that the serum standards or references must be prepared from a base of human serum. The required use of human sera presents severe limitations of availability and cost in making calibrators and controls available. further, turbid sera result after lyophilization and rehydration when triglycerides have been added by conventional methods.

U.S. Patent No. 4,264,471 describes a process for preparing delipified serum or plasma. The process involves first mixing the serum with an anion-cation ion exchange resin to reduce the pH of the serum to 5.2 $\pm$ 0.3 causing the precipitation of lipoproteins from the sera, followed by a separation step in which the supernatant serum is separated from the resin and the precipitated lipoproteins. Thus, this process also removes components from the serum which must be replaced to provide a suitable calibrator or control preparation. As above, turbid sera result after lyophilization and rehydration when triglycerides have been added by conventional methods.

European specification no. 82587A discloses a serum composition and a method of making it which does not require the type of chemical treatment above described. The method provides for an extraction of acid precipitatable molecules to provide a stable, optically clear serum which is suitable for use in making calibrators and controls. This acid precipitation or insolubilization step creates a cloudy or turbid serum which is thereafter clarified by filtration or centrifugation. After this clarification, the serum is stabilized or neutralized by raising the pH to a range of from 7.0 to 8.0. This method does not remove serum components which must be replaced to provide a suitable calibrator or control preparation. Analytes of clinical interest can be introduced in conventional fashion thereafter, either before or after lyophilization of the stabilized, clear serum so prepared. Analytes which can be introduced in such conventional fashion include triglyceride, cholesterol, glucose, various enzymes and ionic analytes of interest. As such, the method disclosed involves the preparation of an optically clear serum of human or animal origin in the absence of added analyte.

In summary, the numerous serum clarification techniques which remove lipids, including triglycerides, from the serum lead those skilled in the art to the conclusion that such serum components cannot withstand the rigorous procedures necessary and must be restored artificially after the procedure has been completed, providing a base serum from which suitable calibrator or control can be prepared. Even European specification no. 82587A does not address the possibility of preparing sera having the desired levels of triglyceride prior to the complete clarification process. As such, there has been no disclosure of a method of achieving this desirable goal or of any product which such a method could offer.

In contrast to the overall teaching and disclosure of the prior art and in accordance with the present invention, we have now found that it is possible to clarify sera of either human or animal sources having added triglycerides, without affecting the observed triglyceride levels. Further, the process of the invention offers sera of acceptable optical clarity in the presence of triglyceride levels which heretofore rendered serum calibrators and controls unreliable or totally unreadable.

According to the present invention, there is provided a method of preparing a stable, optically clear, triglyceride-containing serum from a serum containing one or more components susceptible to precipitation over time, which method comprises the steps in sequence of reducing the pH of the serum to not more than 5, removing insolubles, and then raising the pH to between 7 and 8 to stabilise the serum: characterised in that before removing said insolubles, there is added to the serum one or more triglycerides.

Triglycerides of any of a number of origins can be combined with the serum either before or after the pH reduction step.

The acid insolubilized components are separated from the serum by separation techniques such as filtration or centrifugation and the separated serum is thereafter stabilized by establishing a pH of from 7 to 8 therein. This serum preparation can thereafter be used substantially immediately or can be lyophilized for later use.

Further, in accordance with the invention, there is provided a stable, optically clear, triglyceride-containing serum composition produced by the above described process. This composition retains the highly desirable level of optical clarity even in the presence of serum triglyceride concentrations not previously possible in sera having a clarity sufficient for clinical use. Other clinically significant analytes can be included in the serum in addition to the triglycerides.

One of the significant advantages provided by the invention is that sera of any of a variety of human or animal sources can be used as starting material for preparing calibrators or controls applicable to clinical chemistry evaluations of human serum samples. As such, the sera which can be used in the method can be derived from human sources, such as pooled blood bank sources, or from animal sources, such as bovine, porcine or animal primate sources. These primate and non-primate animal sources are made appropriate for use in clinical chemistry control and standard solutions by the method of the present invention. Other sources of sera which are tested by conventional means and give appropriate results as calibrators or controls are contemplated and can, likewise, be processed in accordance with the method of the invention to provide appropriate compositions.

The triglyceride which is incorporated into the serum by the process of the present invention can be derived from any of a number of sources, including those which are known for use in the preparation of prior art controls and calibrators. For example, chicken egg yolk lipoproteins or water soluble triglycerides, such us trichloroacetate triglyceride, trihemisuccinate glycerol, glycerol triacetate and glycerol tripropionate can be added to the serum. Alternatively, isolated bovine lipoprotein fractions containing triglycerides can be added prior to or after the insolubilization step.

The method in accordance with the invention preferably includes, an additional step of determining the endogenous protein concentration of the serum starting materials, by any conventional protein determination method, since the reproducible accuracy of the triglyceride levels for calibrators and controls is properly compared to a characteristic which defines the concentration of the serum itself. Normal total protein concentrations in serum range from about 6.6 to about 8.3 grams per 100 milliliters of serum in general. However, a variety of recognized methods for determination of protein in serum have been developed, each of which gives varying results on samples of similar protein content. Any standardized method which is used consistently is appropriate and such methods are discussed in Henry, et al, (Eds), Clinical Chemistry, Harper & Rowe, N. Y., pp. 405-502 (1974).

In accordance with the invention, triglyceride is usually added to the serum to an extent sufficient to provide a final triglyceride concentration of 14 milligrams of triglyceride per gram of endogenous protein. In preferred embodiments, sufficient triglyceride is added to provide a normal human triglyceride concentration of 33 milligrams per gram of endogenous protein, and especially preferred is the range of from 35 to 43 milligrams or more of triglyceride per gram of endogenous protein as a final concentration. Such are based on methods which provide at least a normal serum protein concentration range, of from 14 to 17 grams per deciliter of serum.

The insolubilization step is effected by reducing the pH of the serum below about pH 5, preferably by the addition of an inorganic or organic acid which does not interfere with any of the properties of the serum which will affect its intended use. Suitable inorganic acids include hydrochloric, sulfuric, nitric and phosphoric acids. Suitable organic acids include acetic, benzoic, oxalic, phthalic and maleic acids. The reduction of serum pH is preferably effected by the intermittent addition of small amounts of acid with a continuously monitoring pH meter in place.

The serum can be combined with the triglyceride sources either prior to or after establishing a pH in the serum of below 5. In either case, an additional clinically significant substance can be added to the serum along with the triglyceride at this stage or at subsequent stages of the process without interference to their properties or concentrations. Such other substances can include, for example, albumin, calcium, chloride, cholesterol, glucose, inorganic phosphate, iron, potassium, sodium and urea. A more comprehensive list is set forth in the embodiments described by the working example.

In combination with any of the above embodiments, the step of separating the acid insolubilized components from the serum can be performed by known techniques for separating precipitative materials, including filtration, centrifugation and other such mechanical separation techniques. Filtration can be accomplished by use of filtering media or paper, such as asbestos-cellulose or a fiberglass filter media. The addition of diatomaceous earth as a filtering aid has also been found advantageous.

The step of stabilizing the separated serum is accomplished by establishing a pH of from about 7.0 to about 8.0 therein by the addition of am inorganic or organic base which does not interfere with any of the properties of the serum which will affect its intended use. Suitable inorganic bases include sodium, potassium and ammonium hydroxide and metal oxides such as aluminum and copper oxide. Suitable organic bases include trihydroxy aminomethane, tetramethyl ammonium hydroxide and trimethyl amine.

The stabilized, optically clear, triglyceride-containing serum so prepared can be used immediately, stored for later use or lyophilized for long term storage and reconstituted prior to use. Alternatively, the serum can be lyophilized prior to stabilization and stabilized after reconstitution and just prior to use.

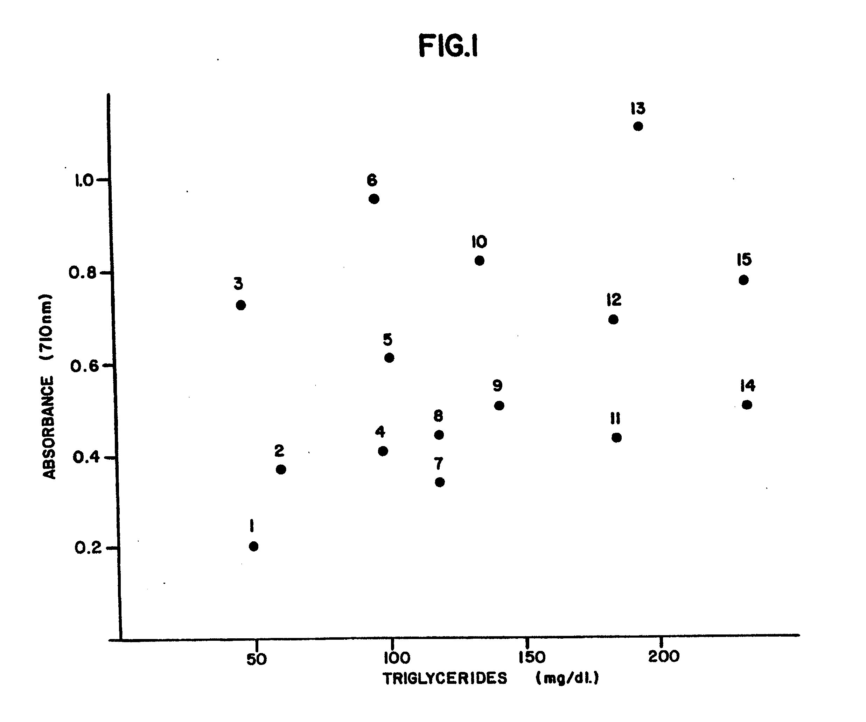

The following working Examples describe experiments which were performed in developing the present invention. Standard commercially available reagent grade chemicals were used whenever possible. Reference will be made to the accompanying drawing which is a graphical illustration of the triglyceride concentration and absorbence of commercial products and the preparations in accordance with the invention as prepared in the Example.

## EXAMPLE

One hundred liters of Plasma Diagnostic Base concentrated bovine serum (Armour Pharmaceutical Co., Tarrytown, NY) were assayed for total protein (T. Protein) concentration on a SMAC continuous flow analyzer (Technicon Instruments Corp., Tarrytown, NY ). A concentration of 19 grams per deciliter (g/dl) was found. The 100 liters of serum was combined with 66.5 g egg yolk triglycerides to give 35 milligrams (mg) triglycerides-per-g of T. Protein. The egg yolk triglycerides were prepared by combining 1 volume of chicken egg yolks with 3 volumes of 50 millimolar (mM) TRIS-succinate buffer (pH 7.5) followed by centrifugation for 1 hour at 5000 g. TRIS is tris (hydroxymethyl) aminomethane. The supernatant was saved and assayed for triglycerides on a SMAC analyzer. A value of 4500 mg triglycerides per dl of the preparation was found.

The serum triglycerides preparation so obtained was stirred until homogenous, about 20 minutes, and 3 molar (M) hydrochloric acid was added to make the solution pH 4.5 (range 4.4 - 4.6). The pH was continuously monitored by a pH meter with the electrode in-dwelling in the preparation. The serum triglycerides preparation was again stirred for 30 minutes. Then, 500 g of Standard SuperCel brand diatomaceous earth (Johns-Mansville, Celite Division, Denver, CO) was added and the preparation stirred for an additional 30 minutes before being filtered. An AMF Cuno (AMF, Cuno Division, Meridian, CT) filter housing containing AMF Cuno 50S filter media was used to remove the diatomaceous earth. The filtered serum was titrated to pH 7.5 (range 7.4 - 7.6) with 4 M sodium hydroxide. The serum was then sterilized by filtering through an autoclaved filter consisting of AMF Cuno 90S media in a AMF Cuno housing. The yield was 97 liters of the triglyceride containing serum preparation, which had a T. Protein of 17.2 g/dl and triglycerides of 602 mg/dl.

This serum was then used to make three separate preparations having levels of other analytes which were the same as those in the TQC Alert 1 Chemistry Control, Chemistry Calibrator and TQC Alert 2 products. Complete information on the additional analytes and their concentrations is available for each of the products to which these preparations were conformed from Technicon Instruments Corp., Tarrytown, NY. The desired levels of other analytes were established by dilution and/or additions to the serum in a conventional manner. The serum triglycerides/T. Protein ratio was approximately 35 mg/g for each.

The formulated serum was filtered through on a Pall NRP 0.2 $\mu$m (micron) filter (Pall Trinity Micro Corp., Cortland, NY) to remove any added microbes. After filtration, the serum was aliquoted into amber vials, frozen and dried under reduced pressure. The lyophilized products were rehydrated with appropriate amounts of diluent (20 ml for calibrator and 10 ml for controls) for use as calibrators for clinical methods or as controls to monitor analysis of analytes of clinical interest.

The above-described preparations were then compared for triglyceride concentration and optical clarity (turbidity) with several of the presently available commercial products offered for use as calibrators and/or controls. The triglyceride concentration of each commercial product and of the preparations in accordance with the invention were measured on a SMAC automated analyzer. Turbidity determinations of each were made by measurement of absorbence at 710 nanometers (nm) using a 1.0 centimeter (cm) light path on a Cary Model 219 spectrophotometer (Varian Associates, Palo Alto, CA). The commercial products and preparations in accordance with the invention which were so tested are grouped by their approximate triglyceride concentrations in Table 1.

## TABLE 1

### 50 mg/dl triglycerides
1. SeraChem Abnormal Control Serum
   (Fisher Scientific Co., Orangeburg, NY)
2. SeraChem Normal Control Serum (Fisher)
3. Monitrol I Chemistry Control
   (American Dade, American Hospital Supply Corp., Miami, FL)

### 100 mg/dl triglycerides
4. Chemistry Calibrator (Technicon Instruments Corp., Tarrytown, NY)
5. Abnormal Control Serum (Ortho Diagnostics, Inc., Raritan, NJ)
6. Normal Control Serum (Ortho)

### 120 mg/dl triglycerides
7. TQC Alert 1 Chemistry Control – Disclosed Process
8. TQC Alert 1 Chemistry Control (Technicon)

### 140 mg/dl triglycerides
9. B – Calibrator (Technicon)
10. Automated Reference Serum (Ortho)

### 180 mg/dl triglycerides
11. Chemistry Calibrator – Disclosed Process
12. Chemistry Calibrator (Technicon)
13. Monitrol II Chemistry Control (American Dade)

### 200 mg/dl triglycerides
14. TQC Alert 2 – Disclosed Process
15. TQC Alert 2 (Technicon)

The results obtained using each of the preparations set forth in Table 1 is graphically illustrated in the accompanying drawing. As can be seen from reference to the drawing, the preparations in accordance with the invention were substantially less turbid than any of the prior art products at each of the triglyceride concentrations tested. This enhanced optical clarity improves the reliability of the preparation at each of the triglyceride concentrations tested and makes it possible to obtain accurate results from sera containing higher concentrations of triglyceride than could reliably be used in the past.

## Claims

1. A method of preparing a stable, optically clear, triglyceride-containing serum from a serum containing one or more components susceptible to precipitation over time, which method comprises the steps in sequence of reducing the pH of the serum to not more than 5, removing insolubles, and then raising the pH to between 7 and 8 to stabilise the serum; characterised in that before removing said insolubles, there is added to the serum one or more triglycerides.

2. A method according to claim 1, wherein said one or more triglycerides are added to the serum before the pH of the serum is reduced to not more than 5.

3. A method according to claim 1, wherein said one or more triglycerides are added to the serum after the pH of the serum has been reduced to not more than 5.

4. A method according to claim 1,2 or 3, which further comprises the step of introducing into said serum an additional clinically significant substance which is or provides albumin, calcium, chloride, cholesterol, glucose, inorganic phosphate, iron, potassium, sodium or urea.

5. A method according to claim 1,2,3 or 4, which comprises the additional step of determining the endogenous protein concentration of the serum; and then combining with said serum at least 14 milligrams of one or more triglycerides, per gram of endogenous protein.

6. A method according to any preceding claim, which includes the additional step of establishing a predetermined endogenous protein concentration in said serum, preferably after stabilizing the separated serum.

7. A method according to claim 6, wherein an endogenous serum protein concentration of from 14 to 17 grams per deciliter of serum is established.

8. A method according to any of claims 1 to 7, which includes the additional step of lyophilizing said separated, stabilized serum.

9. A stable, optically clear, triglyceride-containing serum composition which comprises a serum incorporated with at least 14 milligrams of triglyceride per gram of endogenous protein and having an absorbence which is less than 0.3, made by the method of claim 5.

10. A stable, optically clear, triglyceride-containing serum composition according to claim 9, which comprises serum incorporated with at least 35 milligrams of triglyceride per gram of endogenous protein and has an absorbence which is 0.5 or less.


**Revendications**

1. Procédé de préparation d'un sérum stable, non turbide et contenant des triglycérides, à partir d'un sérum contenant un ou plusieurs composants susceptibles d'être précipités à terme, ledit procédé comprenant successivement les phases d'abaissement du pH du sérum à pas plus de 5, d'élimination des substances insolubles, puis d'augmentation du pH entre 7 et 8 pour stabiliser le sérum, caractérisé en ce que l'on ajoute au sérum un ou plusieurs triglycérides avant d'éliminer lesdites substances insolubles.

2. Procédé selon la revendication 1, caractérisé en ce que l'on ajoute au sérum un ou plusieurs triglycérides avant d'abaisser le pH du sérum à pas plus de 5.

3. Procédé selon la revendication 1, caractérisé en ce que l'on ajoute au sérum un ou plusieurs triglycérides après avoir abaissé le pH du sérum à pas plus de 5.

4. Procédé selon l'une des revendications 1, 2 ou 3, catérisé en ce qu'il comprend en outre la phase consistant à introduire dans ledit sérum une substance supplémentaire cliniquement significative, qui est ou fournit de l'albumine, du calcium, un chlorure, du cholestérol, du glucose, un phosphate inorganique, du fer, du potassium, du sodium ou de l'urée.

5. Procédé selon l'une des revendications 1, 2, 3 ou 4, caractérise en ce qu'il comprend la phase supplémentaire consistant à déterminer la concentration de protéines endogènes du sérum, puis à combiner audit sérum au moins 14 milligrammes d'un ou plusieurs triglycérides par gramme de protéine endogène.

6. Procédé selon l'une quelconque des revendications précédentes, caractérisé en ce qu'il comprend la phase supplémentaire consistant à établir une concentration de protéines endogènes prédéterminée dans ledit sérum, de préférence après avoir stabilisé le sérum séparé.

7. Procédé selon la revendication 6, caractérisé en ce qu'on établit une concentration de protéines endogènes de sérum de 14 à 17 grammes par décilitre de sérum.

8. Procédé selon l'une quelconque des revendications 1 à 7, caractérisé en ce qu'il comprend la phase supplémentaire consistant à lyophiliser ledit sérum séparé et stabilisé.

9. Composition de sérum stable, non turbide et contenant des triglycérides, caractérisée en ce qu'elle comprend un sérum contenant au moins 14 milligrammes de triglycérides par gramme de protéine endogène et présentant une absorbance inférieure à 0,3, préparé au moyen du procédé selon la revendication 5.

10. Composition de sérum stable, non turbide et contenant des triglycérides selon la revendication 9, caractérisée en ce qu'elle comprend un sérum contenant au moins 35 milligrammes de triglycérides par gramme de protéine endogène et présentant un pouvoir d'absorption de 0,5 ou moins.

**Ansprüche**

1. Verfahren zur Herstellung eines stabilen, optisch klaren, Triglyceride enthaltenden Serums aus einem Serum, das eine Komponente oder mehrere Komponenten enthält, die über längere Zeit leicht ausfällt bzw. ausfallen, wobei man in Stufen nacheinander den pH-Wert des Serums auf nicht über 5 erniedrigt, Unlösliches abtrennt und anschließend den pH-Wert auf einen Wert zwischen 7 und 8 erhöht, um das Serum zu stabilisieren,
   **dadurch gekennzeichnet,**
   daß das Serum vor der Abtrennung des Unlöslichen mit einem Triglycerid oder mehreren Triglyceriden versetzt wird.

2. Verfahren gemäß Anspruch 1, wobei das Triglycerid bzw. die Triglyceride dem Serum zugesetzt werden, bevor der pH-Wert des Serums auf nicht über 5 erniedrigt wird.

3. Verfahren gemäß Anspruch 1, wobei das Triglycerid bzw. die Triglyceride dem Serum zugesetzt werden, nachdem der pH-Wert des Serums auf nicht über 5 verringert worden ist.

4. Verfahren gemäß einem der Ansprüche 1, 2 oder 3, das außerdem die Stufe des Versetzens des Serums mit einer weiteren klnisch bedeutsamen Substanz umfaßt, die Albumin, Calcium, Chlorid, Cholesterin, Glucose, anorganisches Phosphat, Eisen, Kalium, Natrium oder Harnstoff ist oder liefert.

5. Verfahren gemäß einem der Ansprüche 1, 2, 3 oder 4, das die zusätzliche Stufe der Bestimmung von endogenem Protein im Serum und des darauffolgenden Versetzens des Serums mit mindestens 14 Milligramm eines Triglycerids oder mehrerer Triglyceride je Gramm endogenen Proteins umfaßt.

6. Verfahren gemäß einem der vorhergehenden Ansprüche, das die zusätzliche Stufe der Einstellung einer vorbestimmten Konzentration an endogenem Protein in dem Serum, vorzugsweise nach der Stabilisierung des abgetrennten Serums, umfaßt.

7. Verfahren gemäß Anspruch 6, wobei eine Konzentration an endogenem Serumprotein von 14 bis 17 Gramm je Deziliter Serum eingestellt wird.

8. Verfahren gemäß einem der Ansprüche 1 bis 7, das die zusätzliche Stufe der Lyophilisierung des abgetrennten, stabilisierten Serums umfaßt.

9. Stabile, optisch klare, Triglyceride enthaltende Serumzusammensetzung, die ein Serum enthält, das mit mindestens 14 Milligramm Triglycerid je Gramm endogenen Proteins versetzt ist und eine Absorption von unter 0,3, bestimmt nach dem Verfahren gemäß Anspruch 5, aufweist.

8

10. Stabile, optisch klare, Triglyceride enthaltende Serumzusammensetzung gemäß Anspruch 9, die ein Serum enthält, das mit mindestens 35 Milligramm eines Triglycerides je Gramm endogenen Proteins versetzt ist und eine Absorption von 0,5 oder darunter aufweist.

FIG.I

ABSORBANCE (710nm)

TRIGLYCERIDES (mg/dl.)

EP 0 140 495 B1